# EUROPÄISCHE PATENTSCHRIFT

(11) **EP 1 196 178 B1**
(45) Veröffentlichungstag und Bekanntmachung des Hinweises auf die Patenterteilung: **09.06.2004**
(21) Anmeldenummer: 00912392.8
(22) Anmeldetag: 24.02.2000
(51) Int. Cl.: A61K 31/713, A61K 31/711, A61P 37/04

(54) **KOVALENT GESCHLOSSENES NUKLEINSÄUREMOLEKÜL ZUR IMMUNSTIMULATION**
COVALENTLY CLOSED IMMUNOSTIMULATING NUCLEIC ACID MOLECULE
MOLECULE D'ACIDE NUCLEIQUE FERMEE PAR COVALENCE POUR L'IMMUNOSTIMULATION

(30) Priorität: 27.07.1999 DE 19935756
(43) Veröffentlichungstag der Anmeldung: 17.04.2002
(73) Patentinhaber: Mologen AG, 14195 Berlin (DE)
(72) Erfinder: JUNGHANS, Claas, D-10589 Berlin (DE); WITTIG, Burghardt, D-14197 Berlin (DE); KÖNIG MEREDIZ, Sven, D-10787 Berlin (DE); SCHROFF, Matthias, D-14057 Berlin (DE)
(74) Vertreter: Omsels, Hermann-Josef
(86) Internationale Anmeldenummer: PCT/DE2000/000565
(87) Internationale Veröffentlichungsnummer: WO 2001/007055

(56) Entgegenhaltungen:
- EP-A- 0 855 184
- WO-A-98/18810
- WO-A-98/21322
- FR-A- 2 732 971
- CLUSEL C ET AL: "EX VIVO REGULATION OF SPECIFIC GENE EXPRESSION BY NANOMOLAR CONCENTRATION OF DOUBLE-STRANDED DUMBBELL OLIGONUCLEOTIDES" NUCLEIC ACIDS RESEARCH,GB,OXFORD UNIVERSITY PRESS, SURREY, Bd. 21, Nr. 15, 25. Juli 1993 (1993-07-25), Seiten 3405-3411, XP000572382 ISSN: 0305-1048
- KRIEG A M ET AL: "OLIGODEOXYNUCLEOTIDE MODIFICATIONS DETERMINE THE MAGNITUDE OF B CELL STIMULATION BY CPG MOTIFS" ANTISENSE & NUCLEIC ACID DRUG DEVELOPMENT,US,MARY ANN LIEBERT, INC., NEW YORK, Bd. 6, Nr. 2, 1996, Seiten 133-139, XP000610233 ISSN: 1087-2906
- ERIE D ET AL: "A DUMBBELL-SHAPED, DOUBLE-HAIRPIN STRUCTURE OF DNA: A THERMODYNAMICINVESTIGATION" BIOCHEMISTRY, 1987, XP002027687

### SEQUENCE LISTING

<110> Mologen Forschungs-, Entwicklungs- und Vertriebs G

<120> Kovalent geschlossenes Nukleinsäuremolekül zur Immunstimulation

<130> Mologen-ImmunPCT

<140>
<141>

<150> DE 199 35 756.0
<151> 1999-07-27

<160> 11

<170> PatentIn Ver. 2.1

<210> 1
<211> 60
<212> DNA
<213> Artificial Sequence

<220>
<223> Description of Artificial Sequence: circular single-stranded with stem loop structure (dumbbell), all phosphodiester

<400> 1
   cttccttgta ctaaccttgc ctcaaggaag gttgatcttc ataacgttgc ctagatcaac 60

<210> 2
<211> 60
<212> DNA
<213> Artificial Sequence

<220>
<223> Description of Artificial Sequence: circular single-stranded with stem loop structure (dumbbell), all phosphodiester

<400> 2
   accttccttg tactaacgtt gcctcaagga aggttgatct tcataacgtt gcctagatca 60

<210> 3
<211> 30
<212> DNA
<213> Artificial Sequence

<220>
<223> Description of Artificial Sequence: linear single-stranded sequence, first five and last three phosphoester linkages by thioate

<400> 3
   gttgcctaga tcaacgttcc ttgtactaac 30

<210> 4
<211> 30
<212> DNA
<213> Artificial Sequence

<220>
<223> Description of Artificial Sequence: linear single-stranded sequence, all phosphodiester

<400> 4
   tcattggaaa acgttcttcg gggcgttctt 30

<210> 5
<211> 30
<212> DNA
<213> Artificial Sequence

<220>
<223> Description of Artificial Sequence: linear single-stranded sequence, first five and last three phosphoester linkages by thioate

<400> 5
   tcattggaaa acgttcttcg gggcgttctt 30

<210> 6
<211> 82
<212> DNA
<213> Artificial Sequence

<220>
<223> Description of Artificial Sequence: circular single-stranded with stem loop structure (dumbbell), all phosphodiester

<400> 6
   cctaggggtt accacctaac gttcttcggg aggtggtaac ccctaggggt taccacctaa 60
   cgttcttcgg gaggtggtaa cc 82

<210> 7
<211> 114
<212> DNA
<213> Artificial Sequence

<220>
<223> Description of Artificial Sequence: circular single-stranded with stem loop structure (dumbbell), all phosphodiester

<400> 7
   tcttcggggc gttctttact aggtcctctc caggttacca cctaagaacg ccccgaagaa 60
   cgttttccaa tgatactagg tcctctccag gttaccacct tcattggaaa acgt 114

<210> 8
<211> 68
<212> DNA
<213> Artificial Sequence

<220>
<223> Description of Artificial Sequence: circular single-stranded with stem loop structure (dumbbell), all phosphodiester

<400> 8
   tcttcggggc gttctttttt aagaacgccc cgaagaacgt tttccaatga tttttcattg 60
   gaaaacgt                                                          68

<210> 9
<211> 116
<212> DNA
<213> Artificial Sequence

<220>
<223> Description of Artificial Sequence: circular single-stranded with stem loop structure (dumbbell), all phosphodiester

<400> 9
   cctaggggtt accaccttca ttggaaaacg ttcttcgggg cgttcttagg tggtaacccc 60
   taggggttac caccttcatt ggaaaacgtt cttcggggcg ttcttaggtg gtaacc     116

<210> 10
<211> 48
<212> DNA
<213> Artificial Sequence

<220>
<223> Description of Artificial Sequence: circular single-stranded with stem loop structure (dumbbell), all phosphodiester

<400> 10
   gttcctggag acgttcttag gaacgttctc cttgacgttg gagagaac              48

<210> 11
<211> 60
<212> DNA
<213> Artificial Sequence

<220>
<223> Description of Artificial Sequence: circular single-stranded with stem loop structure (dumbbell), all phosphodiester

<400> 11
   cttccttgta ctaaccttgc ctcaaggaag gttgatcttc ataacgttgc ctagatcaac 60

## Beschreibung

Die Erfindung betrifft Nukleinsäuremoleküle, besonders solche Nukleinsäuremoleküle, welche unmodifizierte CpG-Nukleosidbausteine aufweisen, die immunmodifizierend wirken können zur Modulation der Aktivität des menschlichen oder tierischen Immunsystems.

Seit einigen Jahren ist bekannt, dass bestimmte kurze Nukleinsäuresequenzen eine erhebliche physiologische Wirkung aufweisen können, indem sie über bisher unbekannte Mechanismen Effektorzellen des Immunsystems stark stimulieren. Diese immunstimulatorischen Nukleinsäuresequenzen ("ISS") sind nur einige Basen lang und wirken nicht über die Expression von auf ihnen kodierten Proteinen. Die meisten bekannten immunmodifizierenden kurzen Oligodesoxyribonukleotidsequenzen ("ODN") enthalten ein unmethyliertes Cytosin-Guanosin-Motiv (CpG-Motiv) (Krieg AM, Yi AK, Matson S, Waldschmidt TJ, Bishop GA, Teasdale R, Koretzky GA, Klinman DM; CpG motifs in bacterial DNA trigger direct B-cell activation; Nature 1995 Apr 6 374:6522 546-9). Es wird angenommen, dass die Erkennung dieses Motivs durch bisher nicht aufgeklärte Erkennungsmechanismen in der eukaryoten Zelle zu einer Art Notrufmechansimus führt, der eine gegen virale oder bakterielle Erreger gerichtete Reaktion auslöst. Gemäß diesem Erklärungsvorschlag soll die Erkennung von CpG-Motiven, deren Vorkommen im Genom von Eukaryonten gegenüber dem von Prokaryonten erheblich unterdrückt ist, dem höheren Tier als "Warnsignal" dienen. Das Vorhandensein der CpG-Motive signalisiert nach dieser Theorie eine Infektion durch prokaryote Erreger, und deren Erkennung ermöglicht eine Bekämpfung unabhängig von bzw. vor der Ausbildung einer T-Helferzell-vermittelten Immunantwort. Die T-Helferzell-unabhängige Stimulierung und Proliferation von B-Zellen wird dadurch ermöglicht, dass für T-Zell- und B-Zell-Aktivierung notwendige kostimulatorische Signale, vor allem die Sekretion von Zytokinen der zellulären sog. Typ 1-Antwort (Th1-spezifische Zytokine wie Interferon gamma, IL-7, IL-12), durch die CpG-abhängigen Signalwege bereitgestellt werden. Außerdem wird durch ISS die Aktivität von NK-Zellen und Makrophagen erhöht. Die Aktivität der sog. Typ-2 Zytokine (IL-4, IL-10) wird demgegenüber unterdrückt, wahrscheinlich durch einen Antagonismus zwischen Th1- und Th2-Antwort (Seder RA und Paul WE, Acquisition of lymphokine-producing phenotype by CD4+ T cells; Annu Rev Immunol. 1994; 12:635-73).

Die starke Stimulation der zellulären Immunantwort ermöglicht eine Einflußnahme auf Regelkreisläufe, die ohne Eingriff nicht zu einer für den Patienten befriedigenden Immunaktivität führen. Dazu gehört die Induktion einer Antwort gegen "schwache", d.h. im Rahmen der MHC-I-Präsentation nicht aktivierende Antigene, wie z.B. Bruchpunktpeptide aus chromosomalen Translokationen oder mutierten Onkogenen, wie sie bei Tumorerkrankungen häufig auftreten (Melief CJ, Kast WM; T-cell immunotherapy of cancer; Res Immunol 1991 Jun-Aug;142(5-6):425-9; ebenfalls: Pasternak G, Hochhaus A, Schultheis B, Hehlmann R; Chronic myelogenous leukemia: molecular and cellular aspects; J Cancer Res Clin Oncol 1998;124(12):643-60). Ebenfalls erwünscht könnte das Brechen der Toleranz gegenüber Autoantigenen, wie etwa der im Tumorzellen des malignen Melanoms exprimierten und MHC-I-repräsentierten Tyrosinase oder Tyrosinhydroxylase (Weber LW, Bowne WB, Wolchok JD, Srinivasan R, Qin J, Moroi Y, Clynes R, Song P, Lewis JJ, Houghton AN; Tumor immunity and autoimmunity induced by immunization with homologous DNA; J Clin Invest 1998 Sep 15;102(6):1258-64; Surman DR, Irvine KR, Shulman EP, Allweis TM, Rosenberg SA, Restifo NJ; Generation of polyclonal rabbit antisera to mouse melanoma associated antigens using gene gun immunization; Immunol Methods; 1998 May 1;214(1-2):51-62) sein. Ein weiterer, erheblich wichtiger Aspekt ist der Adjuvanzeffekt der ISS bei prophylaktischen Impfungen sowie die Möglichkeit, die Reaktion auf eine bestehende Infektion von einer Typ-2-Antwort auf eine Typ-1-Antwort "umzupolen" und so zur Bekämpfung des Erregers instand zu setzen (Kovarik J, et al. CpG oligodeoxynucleotides can circumvent the Th2 polarization of neonatal responses to vaccines but may fail to fully redirect Th2 responses established by neonatal priming; J Immunol. 1999 Feb 1;162(3):1611-7). Für eine Vielzahl von Erregern ist gezeigt worden, dass der Typ der Immunantwort den Verlauf der Infektion bzw. das Überleben des Patienten entscheidend beeinflußt. Soweit allergische Reaktionen ein Überschießen einer Typ-2-Antwort darstellen, wird auch für solche Indikationen ein therapeutischer Effekt von ISS erwartet.

Es ist beobachtet worden, dass bestimmte CpG-haltige Sequenzen einen die Stimulation durch ISS neutralisierenden Charakter haben, das heißt, das solche Sequenzen den stimulatorischen Effekt der ISS unterdrücken können, wenn sie diesen zugegeben werden (Krieg AM, Wu T, Weeratna R, Efler SM, Love-Homan L, Yang L, Yi AK, Short D, Davis HL; Sequence motifs in adenoviral DNA block immune activation by stimulatory CpG motifs; Proc Natl Acad Sci U S A 1998 Oct 13;95(21):12631-6). Ohne den zugrundeliegenden Mechanismus der Wirkung dieser als neutralisierende CpG ("CpG-N") beschriebenen Sequenzmotive genau aufgeklärt zu haben, implizieren die Autoren der zitierten Veröffentlichung, dass dieser Effekt auf eine Hemmung der ISS-Stimulation beschränkt sei. Soweit der Mechanismus der Immuninduktion durch ISS nicht geklärt ist, kann allerdings nicht ausgeschlossen werden, dass auch andere immunmodifizierende Wirkungen von diesen CpG-N-Motiven ausgehen, die therapeutisch verwertbar wären.

Es gibt mindestens eine Krankheit beim Menschen, den systemischen Lupus E-rythematosus, die sich durch die Nachweisbarkeit von anti-DNA-Antikörpern im Patientenserum auszeichnet und bei der ein etiologischer Zusammenhang mit einer Reaktion auf bakterielle ISS vermutet wird (Krieg AM, CpG DNA: a pathogenic factor in systemic lupus erythematosus?, J Clin Immunol 1995 Nov;15(6):284-92). Für solche und andere Indikationen wäre eine Hemmung des zugrundeliegenden Mechanismus durch CpG-N-Motive vorteilhaft.

Unabhängig von der Aufklärung des zugrundeliegenden Mechanismus ist das Potential der CpG-Sequenzen für die Beeinflussung der Immunantwort erheblich und hat zu einem explosiven Interesse der Wissenschaft an dem Phänomen und den Möglichkeiten seiner Anwendung in Therapie und Prophylaxe von Infektionen, Tumorerkrankungen und Immundefekten geführt.

Die Literatur zu ISS behauptet (s. z.B. WO09818810A1, S. 17, II 29-30) und dies wird durch die vorliegende Erfindung bestätigt (s.u.), dass CpG-haltige, immunstimulatorische Sequenzen eine höhere Wirkung zeigen, wenn sie einzelsträngig vorliegen. Die Verabreichung kurzer, offenkettiger einzelsträngiger ISS-Oligodesoxynukleotide zur Immunmodifikation liegt demnach nahe und ist Gegenstand zahlreicher experimenteller Ansätze zur Behandlung von Infektions-, Tumorund Autoimmunerkrankungen. Allerdings werden offenkettige einzelsträngige Oligodesoxynukleotide von extra- und intrazellulären Exonukleasen sehr schnell abgebaut und sind deshalb *in vivo* schwer einsetzbar. Die genannten Nukleasen haben eine erheblich gesenkte enzymatische Aktivität gegenüber modifizierten Phosphoresterbindungen im Rückgrat der Nukleinsäurepolymere; dies hat dazu geführt, dass Phosphorthioester ("Thioate") oder reduzierte Phosphorverbindungen (Phosphonate) in chiraler oder achiraler Form für solche Anwendungen eingesetzt werden, in denen einzelsträngige Nukleinsäuremoleküle in den Patienten eingebracht werden sollen. Diese modifizierten Verbindungen können durch Festphasensynthese hergestellt werden, z.T. allerdings nur in erheblich aufwendigeren Verfahren im Vergleich mit der klassischen DNA-Amiditsynthese. Diese Verbindungen sind aus der Antisense-Forschung bekannt; in klinischen Studien zu Antisense-Strategien zeigte sich allerdings auch, dass sie erhebliche Nebenwirkungen, vor allem auf das Blutgerinnungssystem und das Komplementsystem, haben (siehe z.B. Sheehan und Lan, Blood 92, 1617-1625 (1998)). Im Zusammenhang mit der Verwendung von Thiophosphorsäurederivaten zum Nukleaseschutz von ISS ist außerdem gezeigt worden, dass die Sequenzen weniger stimulatorische Aktivität zeigen, wenn die eigentlich wirksamen Cytosin-Guanosinreste mit den zur Aktivität notwendigen flankierenden Sequenzen selber geschützt sind (siehe dazu WO 98/18810).

Die Lehre von Gebrauch und Herstellung immunstimmulatorischer, CpGenthaltender ISS ist in der WO 98/18810 sowie den darin zitierten Schriften umfassend erläutert. Ausführlich wird in der WO 98/18810 auf die Notwendigkeit des Schutzes von Oligodesoxynukleotiden von Exonukleasen eingegangen. Es werden mehrere Lösungsvorschläge für das Problem der mangelnden Stabilität *in vivo* genannt, die sich jedoch jeweils ausdrücklich auf einzelsträngige lineare ODN beschränken; so werden Thiophosphatester, Dithiophosphatester oder Phosphonate genannt. Die Möglichkeit der Stabilisierung des ODN durch Ausbildung von Sekundärstrukturen, vor allem einer Haarnadel (sog. Stamm-Schleifen-Struktur, engl. "stem-loop") wird in WO 98/18810 erwähnt. Die Herstellung und der Gebrauch von Phosphothioatoligomeren im Zusammenhang mit immunstimmulatorischen Sequenzen ist in der US 5,663,153, US 5,723,335 sowie in der US 5,856,462 beschrieben.

Eine andere Strategie zum Schutz der einzelsträngigen Sequenzen ist in der US 5,750,669 beschrieben. Dabei werden die Enden des Oligomers mit über 5'-5'- und 3'-3'-Bindungen gekoppelten Nukleosidresten versehen, die den exonukleolytischen Abbau hemmen.

Lineare Oligodesoxynukleotide, welche eine Haarnadel-Struktur am 3'-Ende aufweisen (Hosono et al., Biochim Biophys. Acta 244, 339-344 (1995)) sind aus der Antisense-Forschung bekannt.

Doppelt-haarnadelförmige oder kovalent geschlossene "Hantel"-förmige ODN sind aus experimentellen Ansätzen bekannt, bei denen die Kompetition von Bindungsstellen für DNA-bindende Proteine wie Transkriptionsfaktoren im Mittelpunkt der Untersuchung stand (Lim et al. 1997, Nuc. Acids Res. 25, 575-581; Blumenfeld et al., Nuc. Acids Res. 1993, 21, 3405-3411).

Die Autoren der die gesamte technische Lehre der immunstimulatorischen Sequenzen begründenden Anmeldeschrift WO 98/18810 sprechen in einer Publikation davon, dass zum gegebenen Zeitpunkt die Verwendung isolierter ISS wegen deren Stabilitäts- und Toxizitätsproblemen nicht praktikabel sei und sie in Vektorsequenzen eingebaut werden müßten (Weeratna R, Brazolot Millan CL, Krieg AM, Davis HL; Reduction of antigen expression from DNA vaccines by coadministered oligodeoxynucleotides.Antisense Nucleic Acid Drug Dev 1998 Aug;8(4):351-6).

Zusammenfassend ist also festzustellen, dass ISS-ODN ein erhebliches Potential haben, vorteilhafte therapeutische oder prophylaktische immunologische Ansätze zu ermöglichen, jedoch aufgrund der mangelnden Stabilität oder zu hohen Toxizität der wirksamen einzelsträngigen Moleküle für die Anwendung im humanmedizinischen Bereich verbessert werden müssen.

Ausgehend von diesem Stand der Technik ist es die Aufgabe der vorliegenden Erfindung, geeignete Molekülstrukturen zur Verfügung zu stellen, welche immunstimulatorische oder immunmodifizierende exonukleasestabile Desoxyribonukleotidsequenzen enthalten, wobei auf die aus der Natur bekannten Bausteine der phosphorsäurediesterverknüpften Desoxyribonukleinsäure zurückgegriffen werden soll.

Die Bemühungen hinsichtlich der Herstellung nukleasestabiler ISS-ODN konzentrierte sich in der Vergangenheit hauptsächlich auf die Bereitstellung neuer, besser verträglicher Basenmodifikationen in einzelsträngig-linearen Molekülen. Um zu einer besseren Einschätzung der Bedeutung des Einzelstranganteils in den ODN zu kommen, wurden Experimente durchgeführt, in denen die zu untersuchenden Sequenzen teilweise einzelsträngig, doppelsträngig als zwei lineare hybridisierende Stränge sowie als teilweise doppelsträngiges, kovalent geschlossenes ringförmiges Molekül synthetisiert wurden. Überraschenderweise zeigte sich, dass auch doppelsträngige Moleküle mit der relevanten immunstimulatorischen Sequenz im Doppelstrangbereich eine nennenswerte stimulatorische Wirkung ausübten, obwohl nicht anzunehmen wäre, dass diese Sequenz außerhalb eines stark denaturierend wirkenden Milieus hauptsächlich im Einzelstrang vorliegt. Des weiteren zeigte sich allerdings überraschenderweise, dass Moleküle, welche die stimulatorisch wirkenden Sequenzmotive im Einzelstrang-Schleifenbereich aufwiesen, neben der erwarteten stimulatorischen Aktivität eine sehr hohe Stabilität im Serum aufwiesen, was ihre Verwendung als ISS-ODN nahelegte.

Erfindungsgemäß wird demnach die oben geschilderte Aufgabe dadurch gelöst, dass kurze Desoxyribonukleinsäuremoleküle herstellt werden, welche aus einer teilweise einsträngigen, hantelförmigen, kovalent geschlossenen Folge von Nukleosidresten bestehen und eine oder mehr Sequenzen der Basenfolge N¹N²CGN³N⁴ enthalten, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist. Solche kovalent geschlossenen Desoxyribonukleinsäuremoleküle kann man aus offenkettigen Desoxyribonukleinsäuremolekülen, welche eine in Teilen selbstkomplementäre Sequenz aufweisen und miteinander oder einem zweiten Molekül ein intermediär stabiles Hybrid bilden können, welches einen geschlossenen Doppelstrangbereich mit einer Lücke im Zucker-Phosphatrückgrat aufweist, durch Ligation dieser Lücke im Rückgrat durch ein geeignetes Enzym, etwa die DNA-Ligase aus dem Bakteriophagen T4, gewinnen. Alternativ kann ein erfindungsgemäßes Molekül auch durch intramolekulare Ligation eines Moleküls, welches mindestens zwei selbstkomplementäre Bereiche aufweist, welche durch nur eine Lücke im Phosphatrückgrat getrennt sind, gewonnen werden.

Die gewonnenen Moleküle weisen keine freien 5'- oder 3'- Enden auf und sind somit nicht für Exonukleasen zugänglich.

Immunstimmulation heißt in diesem Zusammenhang, dass die Mittler- und Effektorzellen des Immunsystems, also vor allem die zur Zeit bekannten Thymozyten mit Helferfunktion und die cytotoxischen Thymozyten, B-Zellen und sog. NK (natural killer)-Zellen, Makrophagen und Monozyten sowie dendritische Zellen und ihre Vorläufer, sowie bisher in ihrer Funktion nicht aufgeklärte Zellpopulationen mit Funktionen innerhalb des immunsystems, durch die erfindungsgemäße Verwendung von Nukleinsäuremolekülen zur Proliferation, Migration, Differenzierung oder Aktivität angeregt werden. Ein wesentlicher Aspekt der Immunstimmulation ist es z.B., dass B-Zellen, die zur Proliferation normalerweise ein ko-stimulatorisches Signal von Helferthymozyten benötigen, unter Einwirkung von immunstimmulatorischen Nukleinsäuresequenzen auch ohne kostimmulatorische Signale proliferieren können.

Immunmodifikation oder -modulation heißt in diesem Zusammenhang, dass neben einer generellen Stimulation im oben definierten Sinne auch die Art oder der Charakter einer Immunreaktion beeinflusst wird, sei es, dass eine im Entstehen oder der Reifung begriffene Immunreaktion davon betroffen ist, sei es, dass eine schon etablierte Reaktion in ihrem Charakter verändert wird.

Ein Beispiel dafür ist die Wirkung von CpG-haltigen Oligodesoxynukleotiden auf die Entwicklung der Immunreaktion. Unter der Einwirkung dieser Substanzen schütten u.a. Makrophagen und Monozyten typischerweise Interleukin-12 aus, welches seinerseits die Sekretion von Interferon gamma durch NK-Zellen und Helferthymozyten des cytotoxischen Typs anregt. Interferon gamma seinerseits ist ein Stimulator einer Reihe von Komponenten der zytotoxischen, zellvermittelten Immunantwort, darunter CD8-positive Killerzellen, sowie ein potenter Antagonist der von Interleukin-4 vermittelten Bildung von löslichen Antikörpermolekülen vom Subtyp IgG1.

Gesamteffekt einer solchen Immunmodulation durch ISS wäre die Induktion einer zytotoxischen Immunantwort bei Erregern, auf die der Patient bzw. das Versuchstier ohne Modulation mit einer antikörpervermittelten Antwort reagieren würde. (Immunostimulatory oligodeoxynucleotides promote protective immunity and provide systemic therapy for leishmaniasis via IL-12- and IFN-gamma-dependent mechanisms. Walker et al., Proc Natl Acad Sci USA 1999 Jun 8 96:12 6970-5)

Ein Beispiel für die Immunmodulation im klinischen Zusammenhang ist die erfindungsgemäße Anwendung von ISS im klinischen Bild der Allergie oder atopischen Reaktion. Bestimmte Formen dieser Erkrankung sind dadurch ausgezeichnet, dass die betroffenen Patienten einen erheblich über Normwerte erhöhten Plasmaspiegel von Immunoglobulinen des Typs E (IgE) aufweisen. Dieser erhöhte Spiegel an IgE ist nicht nur ein Symptom der Erkrankung, sondern trägt über die Signalketten der Bindung von IgE an Effektorzellen des Immunsystems und nachfolgende Ausschüttung von Chemokinen und parakrinen Botenstoffen, besonders Histamin, zum klinischen Bild der lokalen oder systemischen Überreaktion entscheidend bei. Eine Modulation dieser immunantwort ist Ziel zahlreicher Forschungsvorhaben. Ein vielversprechender Ansatz ist dabei die Behandlung der Patienten mit immunstimmulatorischen ODN.

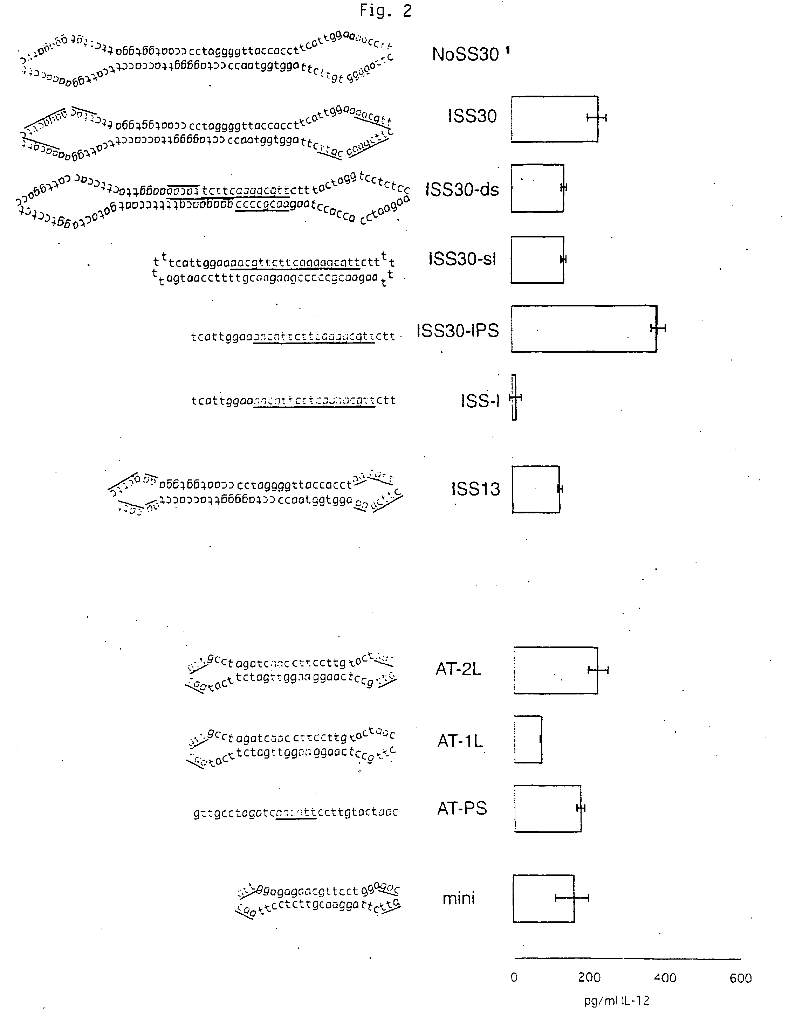

Unter kurzen Desoxyribonukleinsäuremolekülen werden im Sinne der Erfindung solche verstanden, die eine Kettenlänge gemäß den Ausführungsbeispielen (bevorzugt 48 bis 116 Nukleotiden) aufweisen. Nukleinsäuren mit derartigen "kurzen" Kettenlängen sind in den Zeichnungen offenbart (vgl. Abb. 2: die *in vivo* getesteten hantelförmigen Konstrukte NoSS30, ISS30, ISS30-ds, lSS30-sl, ISS13, AT-2L, AT-1L sowie mini).

Die beanspruchten Desoxyribonukleinsäuremoleküle weisen die Sequenz
a) (beispielsweise in der Sequenz "mini" (Abb. 2)) oder
b) (beispielsweise in der Sequenz "AT-2L" (Abb. 2)) auf, oder sie enthalten eine Sequenz der Basenfolge
c)

Vorzugsweise ist Sequenz c) in der Sequenz "ISS30" (Abb. 2) enthalten.

Desoxyribonukleinsäuremoleküle, die aus einer teilweise einsträngigen, hantelförmigen, kovalent geschlossenen Kette von Desoxyribonukleosidresten bestehen und eine oder mehrere Sequenzen der Basenfolge N¹N²CGN³N⁴ enthalten, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist, werden zur Immunstimulation in Menschen oder höheren Tieren verwendet.

Die beanspruchten Desoxyribonukleinsäuremoleküle mit den oben genannten konkreten Sequenzen oder Sequenzbereichen weisen bevorzugt eine Länge von bis zu 200, besonders bevorzugt von 48 bis 116 Nukleotiden auf (vgl. Sequenz ISS 30 (Abb. 2).

Die erfindungsgemäßen Desoxyribonukleinsäuremoleküle werden in einer Form verwendet, bei der die Sequenz der Basenfolge N¹N²CGN³N⁴ bevorzugt im einsträngigen Bereich liegt.

Bei der Verwendung der erfindungsgemäßen Desoxyribonukleinsäuremoleküle kann die Stimulierung *in vitro* oder *in vivo* erfolgen.

Des weiteren werden die erfindungsgemäßen Desoxyribonukleinsäuremoleküle als Adjuvanz in der therapeutischen oder prophylaktischen Vakzinierung verwendet. In diesem Zusammenhang ist auch die Verwendung der Desoxyribonukleinsäuremoleküle zum "Umpolen" einer Typ2-Immunantwort auf eine Typ1-Antwort bevorzugt. Darunter wird der Adjuvanzeffekt der ISS bei prophylaktischen Impfungen verstanden, nämlich die Möglichkeit, die Reaktion auf eine bestehende Infektion von einer Typ-2-Antwort auf eine Typ-1-Antwort "umzupolen" und so zur Bekämpfung des Erregers instand zu setzen.

Weitere vorteilhafte Maßnahmen sind in den übrigen Unteransprüchen beschrieben; die Erfindung wird anhand der folgenden Beispiele näher beschrieben:

### Beispiel 1: Synthese der erfindungsgemäßen Nukleinsäuremoleküle

5'-Phosphorylierte ODN der Sequenz CCTAGGGGTT ACCACCTTCA TTGGAAAACG TTCTTCGGGG CGTTCTTAGG TGGTAACC (TIB-Molbiol, Berlin) wurden 5 min auf 90°C erhitzt und anschließend auf Eis gekühlt, um die Ausbildung der Haarnadelstruktur zu ermöglichen. Selbstkomplementäre Überhänge wurden bei einer Endkonzentration von 1 µg/µl DNA in Gegenwart von T4-DNA Ligase (0,1 U/µg ODN) 24 h bei 37°C ligiert. Nach Phenolextraktion und anschließender Extraktion mit Chloroform sowie Präzipitation mit isopropanol in Anwesenheit von MgCl₂ (f.c. 10 mM) und NaAc (f.c. 300 mM) wurde das Produkt nach Zentrifugation und Aufnehmen in Wasser erhalten.

Zur Reinigung von Endotoxinen wurde das Ligationsprodukt einer anschließenden Anionenaustauschchromatographie (Trägermaterial: LiChrospher DMAE, Merck Darmstadt; 0-1 M NaCl in 50 mM NaPhosphat) unterworfen und durch Isopropanolpräzipitation konzentriert. Für in-vivo-Versuche wird das Verfahren unter sterilen Bedingungen durchgeführt und das Endprodukt in sterilem PBS aufgenommen.

### Beispiel 2: Milzzellengewinnung und Zellkultur, Zytokin-Assays

Milzzellen wurden aus frischen Milzen von 5-10 Wochen alten BALB/c Mäusen (Bomholtgard Breeding & Research Center, Dänemark) gewonnen. Zwei frisch isolierte Milzen wurden mit Hilfe eines 40 µm Metallfilters homogenisiert und die gewonnenen Zellen wurden in 20 ml RPMI 1640 (10% FCS, 100 U/ml Penicillin und 100 µg/ml Streptomycin, Biochrom, Berlin) aufgenommen. Erythrozyten und Plättchen wurden durch Gradientenzentrifugation (Ficoll 1.077; Biochrom, Berlin) entfernt. Die Zellen wurden in einer Endkonzentration von 10⁶ Zellen/ml bei 37°C in einem mit 5% CO₂ gespülten Brutschrank inkubiert.

10⁵ frisch isolierte Milzzellen wurden in 96-Loch-Platten mit den nach Beispiel 1 hergestellten Konstrukten für 24 h inkubiert. Die Konzentration der Konstrukte betrug dabei 2 µM. Zytokine im Überstand wurden durch ELISA (Biosource, Belgium) gemäß den Beschreibungen des Herstellers des ELISAs bestimmt. Für alle Meßpunkte wurden mindestens Dreifachbestimmungen durchgeführt.

Die Ergebnisse dieses Versuchs sind in Abb. 2 gezeigt. Es wurden 6 Konstrukte unterschiedlicher Architektur, aber gleicher ISS untereinander verglichen. Die ISS sind jeweils durch Unterstreichung gekennzeichnet. Das aktivste Molekül ist das Phosphorthorthioat-geschützte, lineare Molekül (ISS30-IPS). Das ungeschützte Molekül (ISS30-I) zeigt dagegen praktisch keine Stimulation der IL-12-Produktion. Die nächsthöchste Wirkung in diesem Assay zeigt das Molekül, welches die ISS in der Schlaufe trägt (ISS30). Das Vergleichskonstrukt ohne ISS (NoSS30) zeigt dagegen keine Wirkung. Liegt die ISS im doppelsträngigen Bereich (lSS30-ds, ISS30-sI), ist die Wirkung demgegenüber deutlich verringert, aber immer noch deutlich über der Kontrolle bzw. dem ungeschützten lineraren Molekül. Es zeigt sich, dass auch kleinere Kontrukte gleicher Molarität Wirkungen in der gleichen Größenordnung haben können (siehe AT-2L). In diesem Fall liegt die gemessene Wirkung sogar über der des phosphorthioatgeschützten, linearen Moleküls. Ein Konstrukt mit nur einer ISS pro Molekül zeigt demgegenüber eine verringerte Wirkung (AT-1L). Letztlich läßt sich die Länge des Moleküls auf eine geringe Minimalgröße reduzieren, ohne den Effekt zu verlieren (siehe mini).

### Beispiel 3: Serumstabilität

5 µg des Desoxyribonukleotids WOT-11-P (Phosphat-GAAGAACGTT TTCCAAT-GAT TTTTCATTGG AAAAC) (TIB Molbiol) wurden mit 75 µCi gamma-32P-ATP (6000 Ci/mmol) (NEN) in Anwesenheit von 10u T4 Polynucleotidekinase (MBI-Fermentas, Leon-Rot) entsprechend den Angaben des Herstellers markiert. Das Enzym wurde durch Erhitzen auf 75 °C während 1 h inaktiviert. Der Ansatz wurde mit Wasser auf 50 µl und durch ZG-50 Größenausschluß-Säule (Pharmacia) gereinigt. Das radioaktiv markierte Molekül wurde mit unmarkiertem, 5'-phosphorylierten WOT-10-P (5'Phosphat-GTTCTTCGGG GCGTTCTTTT TTAAGAACGCCCC) (TIB Molbiol) in Anwesenheit von 1 U T4 DNA Ligase (MBI-Fermentas, Leon-Rot) und 1 mM ATP 2 h bei 37°C umgesetzt. Nichtligierte ODN wurden durch nachfolgende Inkubation mit T7-DNA-Polymerase entfernt. Die Aktivität des erhaltenen Präparats wurde im Szintillationszähler (Beckmann Instruments) zu 78000 cpm/µl, entsprechend 7.800 cpm / ng bestimmt.

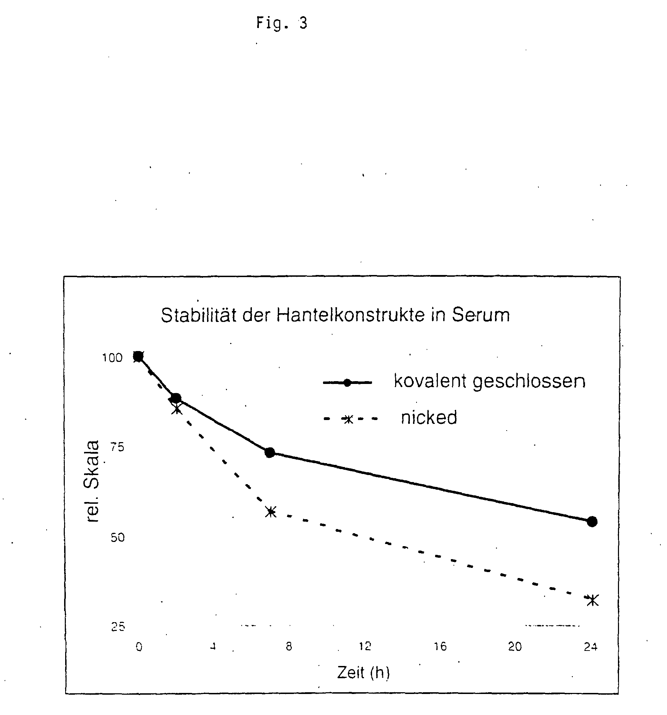

Für die Bestimmung der Stabilität der erhaltenen Konstrukte im Serum wurden 2.5 µl der DNA (195.000 cpm entsprechend 25 ng DNA) mit 20 µl nicht-inaktiviertem Fötalen Kälberserum (Life Technologies), alternativ frisch gewonnenem menschlichen Probandenserum, in 180 µl RPMI Medium (Life Technologies) zugegeben. Alle Bestimmungen wurden dreifach durchgeführt. Die Proben wurden bei 37°C inkubiert; 20 µl Aliquotproben wurden bei 0, 1, 2, 7, 11 und 24 h genommen und bei -40 C aufbewahrt. Je 5 µl der Proben wurden mit 20 µg/ml Proteinase K (Life) während 1h verdaut. Die Proben wurden anschließend denaturierender Polyacrylamidelektrophorese unterworfen, die Gele digitalisiert (Molecular Dynamics) und die Bandenintensität verglichen (IP Labgel). Die Auswertung ist in Abb. 3 gezeigt. Jeder Datenpunkt ist das arithmetische Mittel aus drei Bestimmungen.

### Beispiel 4: Anwendung der Konstrukte in der Maus

Die Konstrukte wurden in vivo getestet. Sechs Wochen alte weibliche BALB/C Mäuse wurden mit je 50 µg des entsprechenden Konstrukts in 250 µl sterilem PBS intraperitoneal injiziert. Es wurden nach 2, 6, 24 und 72 h je 50 µl Blut entnommen, mit Heparin versetzt, abzentrifugiert, und das Serum bei -70°C gelagert. Die Proben wurden gemeinsam auf IL-12 mit ELISA getestet (s.o.). Alle Präparationen wurden mit dem Endotoxin-Assaysystem aus limulus (LAL-Test, BioWhittaker) auf Endotoxin getestet. Alle Proben hatten einen unter der Nachweisgrenze liegenden Endotoxin-Gehalt.

### Beispiel 5: Inkubation von menschlichen periphären Blutmononukleären Zellen (PBMC) mit zirkulären ISS-ODN

PBMC werden aus Blutspenden eines Probanden mit erhöhtem IgE-Spiegel nach bekannten Methoden isoliert und in einer Konzentration von 10⁶ Zellen pro ml in RPMI-Medium (10% fötales Kälberserum (FCS)) inkubiert. Man gibt zu den Proben jeweils
A: nichts (Kontrolle);
B: 1 µg/ml anti-CD-40-Antikörper sowie 5 ng/ml IL-4;
C: 1 µg/ml anti-CD-40-Antikörper, 5 ng/ml IL-4 sowie 2 µM der erfindungsgemäßen ODN ISS30;
D: 2 µM der erfindungsgemäßen ODN ISS30;
und inkubiert die Zellen 10 Tage bei 37° Grad im Brutschrank unter Standard-Zellkulturbedingungen. Anschließend werden die Zellen abzentrifugiert und IgE per ELISA aus dem Überstand bestimmt. Man erhält als Werte (alle Werte in ng IgE pro ml Überstand)

| | A | B | C | D |
|---|---|---|---|---|
| Proband 1 | 9,2 | 10,3 | 3,8 | 4,3 |
| Proband 2 | 4,6 | 6,7 | 1,7 | 3,0 |
| Proband 3 | 1,5 | 3,2 | 0,6 | 0,1 |

### Beispiel 6:

Ein anderer Aspekt der Immunstimmulation ist die verbesserte Reifung von Dendritischen Zellen unter Einwirkung von ISS-ODN. Ein Beispiel ist die Gewinnung von CD14, CD8 und CD4 positiver Zellen

### a) Gewinnung von CD14, CD8 und CD4 positiver Zellen

Dendritische Zellen (DC) CD14 positive Zellen wurden mittels magnetischer Partikel (Milteniy Biotec, Bergisch Gladbach, Germany) isoliert wie vom Hersteller beschrieben. Dazu wurden mononukleäre Zellen aus periphärem Blut gesunder Spender über einen Ficolldichtegradienten (Pharmacia) isoliert und erst mit de für den jeweiligen Oberflächenmarker spezifischen Magnetpartikeln auf den entsprechenden Trennsäulen separiert.

### b) Reifung dendritischer Zellen

CD14-positive Zellen der monozytären Linie wurden in CellGro Medium (Cell Genix, Freiburg) kultiviert, welches Glutamax I(Gibco Life, Karlsruhe) , GM-CSF (800 U/ml, Leucomax 300; Novartis Pharma GmbH) und IL-4 (500 U/ml, R&D Systems GmbH) enthielt. Am Tage drei und fünf wurde jeweils die Hälfte des Mediums gegen frisches Medium ausgetauscht. Am Tag sieben wurde das Medium vollkommen erneuert und zusätzlich mit Prostaglandin E2 (1 µg/ml, Sigma), TNFa (20 ng/ml, Sigma), IL-6 (1000 U/ml, R&D Systems GmbH) und 2 µM der erfindungsgemäßen ISS 30 angereichert.

### c) IFN-gamma ELISpot assay

Nitrozellulose beschichtete Mikrotiterplatten (96 fach) HA S45 (Milipore, Eschborn) wurden bei 4 Grad über Nacht mit Anti-Interferon (IFN) gamma (10 µg/ml, Mabtech) beschichtet und anschließend mit 5% menschlichem Serumalbumin (HSA) geblockt (Baxter, Unterschleißheim). Die Platten wurden gewaschen und anschließend mit 50 µl wie beschrieben gereifter Dendritischer Zellen (DC) (2 x 10⁵/ml) und 50 µl wie beschrieben gewonnenen CD4-positiven T-Zellen (2 x 106/ml) 72 h bei 37 °C und 7 % CO₂ mit 100 µl der entsprechenden Antigene inkubiert. 10 Lf Tetanustoxoid (TT) (Chiron Behring GmbH & Co., Marburg) wurden als Antigene verwendet. Nach ausgiebiegem Waschen wurden die Platten mit IFN gamma spezifischem, biotinyliertem Antikörper aus Maus (5 µg/ml, Mabtech, Germany) inkubiert und dieser mit Avidingekoppelter Alkaliner Phosphatase (Sigma-Aldrich, Steinheim) und nachfolgender Farbreaktion mit BCIP-NBT (Sigma-Aldrich) nachgewiesen. Die Analyse der Spots erfolgte mit computerunterstützter Videoanalyse (KS400 imaging system software, Carl Zeiss, Eching) auf einem Carl Zeiss Vision Microskop Axioplan 2.

Die Anwesenheit von erfindungsgemäßen ISS-ODN bewirkte eine erhebliche Steigerung der Zahl an Spot s im IFN-gamma ELIspot. Zusätzlich steigerte die Anwesenheit von ISS-ODN während der Ko-Inkubation der Dendritischen und T-Helferzellen die Antigenspezifität der Reaktion erheblich; so änderte sich der Quotient von TT-spezifischen (TT anwesend während der Ko-inkubation auf der ELlspot-Platte) zu unspezifischen (kein TT) Spots von 8.9 (mit ISS) zu 2.8 (ohne ISS).

### Kurze Erläuterung der Zeichnungen

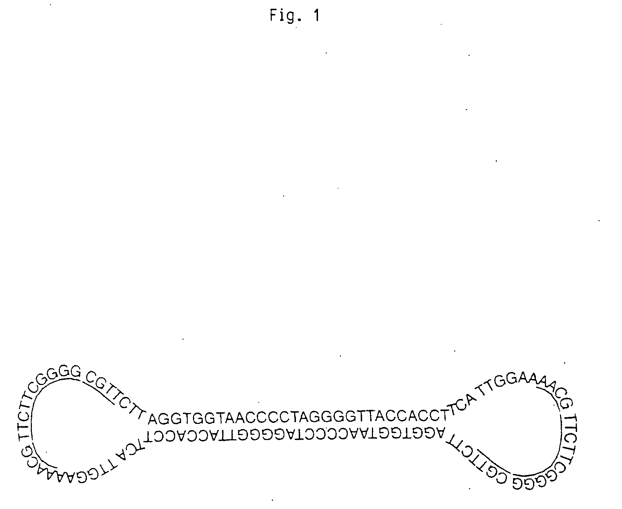

- Abb. 1: zeigt beispielhaft ein erfindungsgemäßes Konstrukt, enthaltend zwei CpG-Motive, schematisch. Die ISS sind unterstrichen dargestellt.
- Abb. 2: zeigt die Ergebnisse der IL-12 Bestimmungen in-vitro in pg IL-12/ml. Daneben sind die verwendeten Kontrukte schematisch dargestellt.
- Abb. 3: zeigt das Ergebnis der Stabilitätsmessung in fötalem Kälberserum in Abhängigkeit der Zeit.

## Patentansprüche

1. Desoxyribonukleinsäuremolekül,
• das aus einer teilweise einsträngigen, hantelförmigen, kovalent geschlossenen Kette von Desoxyribonukleosidresten besteht, und
• eine oder mehr Sequenzen der Basenfolge N¹N²CGN³N⁴ enthält,
• wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist,
**dadurch gekennzeichnet, dass** die Sequenz
a) GTTCCTGGAG ACGTTCTTAG GAACGTTCTC CTTGACGTTG GAGAGAAC oder
b) ACCTTCCTTG TACTAACGTT GCCTCAAGGA AGGTTGATCT TCATAACGTT GCCTAGATCA ist, oder
c) eine Desoxyribonukleinsäuresequenz der Basenfolge AACG TTCTTCGGGG CGTT enthält,
d) und wobei das Desoxyribonukleinsäuremolekül eine Länge von 40 bis 200 Nukleotide aufweist.

2. Desoxyribonukleinsäuremolekül nach Anspruch 1, wobei die Basenfolge aus Merkmal c) in der Sequenz CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC enthalten ist.

3. Desoxyribonukleinsäuremolekül nach Anspruch 1 oder 2, wobei das Desoxyribonukleinsäuremolekül bevorzugt eine Länge von 48 bis 116 Nukleotide aufweist.

4. Desoxyribonukleinsäuremolekül nach einem oder mehreren der Ansprüche 1 bis 3, wobei die Sequenz der Basenfolge N¹N²CGN³N⁴ im einsträngigen Bereich liegt.

5. Verwendung von Desoxyribonukleinsäuremolekülen nach den Ansprüchen 1 bis 4 zur Herstellung eines Arzneimittels oder eines Bestandteils eines Arzneimittels zur Immunstimulation in Menschen oder höheren Tieren.

6. Verwendung nach Anspruch 5 , wobei die Stimulierung *in vitro* oder *in vivo* erfolgen kann.

7. Verwendung nach einem der Ansprüche 5 oder 6 als Adjuvanz in der therapeutischen oder prophylaktischen Vakzinierung.

## Claims

1. Desoxyribonucleic acid molecule
• that consists of a partially single-stranded, dumbbell-shaped, covalently closed chain of desoxyribonucleic residues and
• comprises one or more sequences of the base sequence N¹N²CGN³N⁴
• where N¹N² is an element taken from the group of GT, GG, GA, AT or AA, N³N⁴ is an element taken from the group of CT or TT, and C is desoxycytosine, G is desoxyguanosine, A is desoxyadenosine and T is desoxythymidine,
**characterized by** the fact that the sequence is
a) GTTCCTGGAG ACGTTCTTAG GAACGTTCTC CTTGACGTTG GAGAGAAC or
b) ACCTTCCTTG TACTAACGTT GCCTCAAGGA AGGTTGATCT TCATAACGTT GCCTAGATCA, or
c) contains a desoxyribonucleic acid sequence AACG TTCTTCGGGG CGTT,
d) and in which the desoxyribonucleic acid molecule is of a length of 40 to 200 nucleotides.

2. Desoxyribonucleic acid molecule according to claim 1, where the base sequence from element c) is contained in the sequence CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC.

3. Desoxyribonucleic acid molecule according to claim 1 or 2, where the desoxyribonucleic acid molecule is preferably of a length of 48 to 116 nucleotides.

4. Desoxyribonucleic acid molecule according to one or several of claims 1 to 3, where the sequence comprising the base sequence N¹N²CGN³N⁴ is situated in the single stranded region.

5. Use of desoxyribonucleic acid molecules according to claims 1 to 4 for the production of a pharmaceutical or an ingredient of a pharmaceutical for immunostimulation in human beings or higher animals.

6. Use according to claim 5 , where the stimulation can be effected in vitro or in vivo.

7. Use according to claim 5 or 6 as an adjuvant in therapeutic or prophylactic vaccination.

## Revendications

1. Molécule d'acide désoxyribonucléique
* qui se compose d'une chaîne de groupements désoxyribonucléoside fermée de façon covalente, en forme d'haltère et partiellement monocaténaire, et
* qui contient une ou plusieurs séquences ayant comme séquence de bases N¹N²CGN³N⁴,
* où N¹N² est un élément du groupe GT, GG, GA, AT ou AA, N³N⁴ est un élément du groupe CT ou TT, ainsi que C désoxycytosine, G désoxyguanosine, A désoxyadénosine et T désoxythymidine,
**caractérisée en ce que** la séquence est
a) GTTCCTGGAG ACGTTCTTAG GAACGTTCTC CTTGACGTTG GAGAGAAC ou
b) ACCTTCCTTG TACTAACGTT GCCTCAAGGA AGGTTGATCT TCATAACGTT GCCTAGATCA, ou
c) contient une séquence d'acide désoxyribonucléique de la séquence de bases AACG TTCTTCGGGG CGTT,
d) et dans laquelle la molécule d'acide désoxyribonucléique présente une longueur comprise entre 40 et 200 nucléotides.

2. Molécule d'acide désoxyribonucléique selon la revendication 1, dans laquelle la séquence de bases de la caractéristique c) est contenue dans la séquence CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC.

3. Molécule d'acide désoxyribonucléique selon la revendication 1 ou 2, dans laquelle la molécule d'acide désoxyribonucléique possède de préférence une longueur comprise entre 48 et 116 nucléotides.

4. Molécule d'acide désoxyribonucléique selon une ou plusieurs des revendications 1 à 3, dans laquelle la séquence de la séquence de bases N¹N²CGN³N⁴ se trouve dans la zone monocaténaire.

5. Utilisation de molécules d'acide désoxyribonucléique selon les revendications 1 à 4 pour fabriquer un médicament ou un constituant d'un médicament destiné à l'immunostimulation chez les hommes ou les animaux supérieurs.

6. Utilisation selon la revendication 5, dans laquelle la stimulation peut être réalisée *in vitro* ou *in vivo.*

7. Utilisation selon l'une des revendications 5 ou 6 en tant qu'adjuvant dans la vaccination thérapeutique ou prophylactique.
